(19) Europäisches Patentamt
European Patent Office
Office européen des brevets

(11) **EP 1 857 112 B1**

(12) **EUROPEAN PATENT SPECIFICATION**

(45) Date of publication and mention
of the grant of the patent:
**15.05.2013 Bulletin 2013/20**

(21) Application number: **06715426.0**

(22) Date of filing: **09.03.2006**

(51) Int Cl.:
*A61K 31/575* (2006.01)   *A61K 9/00* (2006.01)
*A61K 9/127* (2006.01)   *A23L 1/30* (2006.01)
*A61K 47/24* (2006.01)   *A61P 35/00* (2006.01)
*A61P 35/04* (2006.01)

(86) International application number:
**PCT/JP2006/304555**

(87) International publication number:
**WO 2006/095798 (14.09.2006 Gazette 2006/37)**

(54) **ANTICANCER COMPOSITION COMPRISING LIPOSOMES CONTAINING PHYTOSTEROLS**

MITTEL GEGEN KREBS MIT PHYTOSTEROL-HALTIGEN LIPOSOMEN

COMPOSITION ANTICANCER COMPRENANT DES LIPOSOMES QUI CONTIENNENT DES PHYTOSTEROLS

(84) Designated Contracting States:
**AT BE BG CH CY CZ DE DK EE ES FI FR GB GR HU IE IS IT LI LT LU LV MC NL PL PT RO SE SI SK TR**

(30) Priority: **09.03.2005 JP 2005065119**

(43) Date of publication of application:
**21.11.2007 Bulletin 2007/47**

(73) Proprietor: **SUNSTAR INC.**
**Takatsuki-shi,**
**Osaka 569-1195 (JP)**

(72) Inventors:
• **IMANAKA, Hiromichi**
 **Osaka 5691195 (JP)**
• **ISHIKADO, Atsushi**
 **Osaka 5691195 (JP)**
• **OKU, Naoto**
 **Shizuoka 4240857 (JP)**

(74) Representative: **TBK**
**Bavariaring 4-6**
**80336 München (DE)**

(56) References cited:
WO-A-03/039437    WO-A-2004/010977
WO-A1-03/037360    JP-A- 04 501 858
JP-A- 07 278 016    JP-A- 2002 503 209
JP-A- 2004 507 492    JP-A- 2004 534 793

• SHIMIZU K ET AL: "Targeting of soybean-derived sterylglucoside liposomes to liver tumors in rat and mouse models" BIOLOGICAL & PHARMACEUTICAL BULLETIN (OF JAPAN), PHARMACEUTICAL SOCIETY OF JAPAN, TOKYO, JP, vol. 21, no. 7, 1 January 1998 (1998-01-01), pages 741-746, XP009117233 ISSN: 0918-6158
• AWAD A B ET AL: "In vitro and in vivo (SCID mice) effects of phytosterols on the growth and dissemination of human prostate cancer PC-3 cells" EUROPEAN JOURNAL OF CANCER PREVENTION, LIPPINCOTT WILLIAMS & WILKINS, PHILADELPHIA, US, vol. 10, no. 6, 1 January 2001 (2001-01-01), pages 507-513, XP009117225 ISSN: 0959-8278
• MAITANI Y ET AL: "Application of sterylglucoside-containing particles for drug delivery" CURRENT PHARMACEUTICAL BIOTECHNOLOGY, BENTHAM SCIENCE PUBLISHERS, NL, vol. 6, no. 1, 1 January 2005 (2005-01-01), pages 81-93, XP009117228 ISSN: 1389-2010
• KAWANO K.: 'Development of liver-targeted liposomes entrapping pirarubicin for liver cancer chemotherapy' no. 45, 2003, pages 23 - 29, XP003000045

EP 1 857 112 B1

**Description**

TECHNICAL FIELD

The present invention relates to an orally administered composition for suppressing cancer, comprising liposomes that contain phytosterol, and to the prevention and treatment of cancer using such liposomes.

BACKGROUND ART

Research into the use of microparticulate formulations as drug carriers has been actively pursued in recent years as one method for achieving dosage form modifications. One area of this research involves investigations into the utilization of liposomes as drug carriers for oral administration. The ability of liposomes to incorporate watersoluble drugs in their internal aqueous layer and lipid-soluble drugs in their lipid bilayer has led to efforts to apply liposomes as a drug carrier in drug delivery system (DDS) formulations whose goals are, inter alia, targeting of the drug, sustained release, and a lessening of side effects. It is also known that liposomes provide a high level of biosafety because they are constructed from the components of biological membranes. Insulin, which generally has exhibited almost no therapeutic efficacy by oral administration, has been found to exhibit an increased therapeutic efficacy by oral administration when enclosed in modified liposomes (Nonpatent document 1, 2 and 3). It has also been reported that the bioavailability of erythropoietin is increased by its insertion in a liposome (Nonpatent document 4). However, in these technologies the therapeutic efficacy has been based on the component incorporated in the liposome and the liposome has been used simply as a carrier. Very little attention has thus been paid to the therapeutic efficacy of the liposome itself.

Sterols are a constituent component of plant and animal cells; the sterols present in animal cells are known as animal sterol and the sterols present in plant cells are known as phytosterol (and also as plant sterol). Cholesterol is a representative example of the animal sterol, and its ingestion in excess is known to have a negative effect on health. On the other hand, research on the phytosterol has very recently become quite active, and it has been reported that phytosterol have the ability to lower plasma cholesterol and the ability to inhibit the appearance of colorectal cancer.

Nonpatent document 1: Hirofumi Takeuchi et al., "Enteral Absorption of Insulin in Rats from Micoadhesive Chitosan-Coated Liposanes", Pharmaceutical Research, 13(6), 896-901, 1996

Nonpatent document 2: Muramatsu, K. et al., *Dipalmitoylphosphatidylcholine Liposomes with Soybean-Derived Sterols and Cholesterol as a Carrier for the Oral. Administration of Insulin in Rats", Biological & Pharmaceutical Bulletin, 19(8), 1055-1058, 1996

Nonpatent document 3: Kazunori Iwanaga at al., "Application of surface-coated liposomes for oral delivery of peptide: Effects of coating the liposome's surface on the GI transit of insulin", Journal of Pharmaceutical Sciences, 88, 248-252, 1999

Nonpatent document 4: Yoshie Maitani et al., "Oral administration of recombinant human erythropoietin in liposomes in rats: Influence of lipid composition and size of liposomes on bioavailability" Journal of Pharmaceutical Sciences, 85, 440-445, 1996.

WO 2004/010977 A is directed to a method for decreasing the variability in the bioavailability of an orally administered drug, i.e. for drugs used in anti-cancer chemotherapy. In the technology described in this document liposomes are used in order to improve the variability of the bioavailability of different drugs. The liposomes may also contain phytosterols.

WO 03/039437 A is directed to pharmaceutical preparations comprising the anti-cancer drug "paclitaxel" encapsulated in liposomes which may comprise steroids.

SHIMIZU K ET AL; BIOLOGICAL & PHARMACEUTICAL BULLETIN, PHARMACEUTICAL SOCIETY OF JAPAN, TOKYO, JP, vol. 21, no. 7, pages 741-746, describes liposomes comprising the lecithin dipalmitoylphosphatidylcholin (DPPC) and soybean-derived sterylglucosides (SG) entrapping doxorubicin (DOX).

AWAD A B ET AL; EUROPEAN JOURNAL OF CANCER PREVENTION, LIPPINCOTT WILLIAMS & WILKINS, PHILADELPHIA, US, vol. 10, no. 6, pages 507-513, describes the *in vitro* and *in vivo* effects of phytosterols on the growth and the dissemination of human PC-3 cells.

MAITANI Y ET AL; CURRENT PHARMACEUTICAL BIOTECHNOLOGY, BENTHAM SCIENCE PUBLISHERS, NL, vol. 6, no. 1, pages 81-93, describes sterylglucoside-containing particles for drug delivery. As an example for such particles liposomes are mentioned, which are composed of dipalmitoylphosphatidylcholin (DPPC) and cholesterine as well as soybean-derived sterylglucoside (SG).

DISCLOSURE OF THE INVENTION

PROBLEMS TO BE SOLVED BY THE INVENTION

However, there have been problems with regard to phytosterol with the development of methods of administration and carriers because pbytosterol is insoluble or poorly soluble in water. The present invention therefore takes as an object the introduction of a phytosterol-containing orally administered composition that suppresses cancer. An additional object of the present invention is the introduction of a method for preventing or treating cancer that uses said Liposomes.

MEANS FOR SOLVING THE PROBLEMS

The present inventor carried out intensive investigations in order to achieve these objects and discovered as a result that liposome that utilizes phytosterol as a constituent component of the liposome membrane has the ability to inhibit cancer metastasis and that liposome that contains lecithin and β-sitosterol as constituent components of the liposome membrane has a particularly strong ability to inhibit cancer metastasis. Accordingly, the present invention relates to the application of phytosterol-containing liposome to prevent or treat cancer.

That is, the present invention provides a use in accordance with the following items.

Item 1. Phytosterol for use in the prevention or treatment of cancer, wherein the phystosterol is contained in a liposome.

Item 2. The phytosterol for use according to item 1, wherein the phytosterol is at least one selected from the group consisting of β-sitosterol, campesterol, stigmasterol, brassicasterol, ergosterol, and ergostadienol.

Item 3. The phytosterol for use according to item 1, wherein the phytosterol content of the liposome is 1 to 33 weight%.

Item 4. The phytosterol for use according to any of items 1 to 3, wherein the anticancer activity is an inhibition of cancer metastasis.

Item 5. The phytosterol for use according to any of items 1 to 4, wherein the amount of liposome that is orally administered is 10 mg to 10000 mg per adult per day.

Item 6. The phytosterol for use acccording to any of items 1 to 5, wherein the orally administered composition is a food composition.

Item 7. The phytosterol for use according to any of items 1 to 5, wherein the orally administered composition is a pharmaceutical composition.

BRIEF DESCRIPTION OF THE DRAWINGS

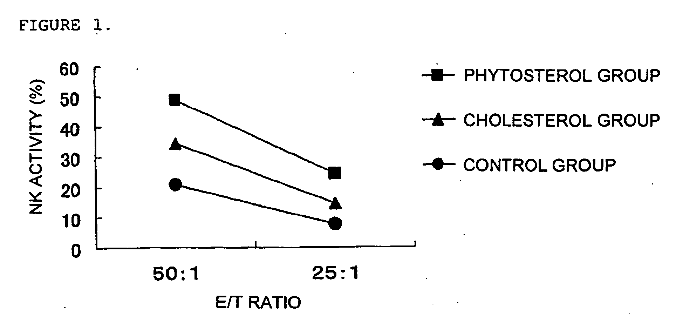

Figure 1 is a graph that shows the test results for NK activity. The vertical axis shows NK activity (%), while the horizontal axis shows the effector cell : target cell ratio (E/T ratio 50 : 1, 25 : 1). The filled circles show the control group; the filled triangles show the cholesterol group; and the filled squares show the phytosterol group.

The orally administered composition of the present invention acts to inhibit cancer metastasis and contains as its effective component liposome that contains phytosterol as a constituent component of the membrane. This capacity to inhibit cancer metastasis referenced by the present invention encompasses the inhibition of a developed cancer into another tissue or organ. The method of the present invention for preventing and treating cancer comprises the oral administration of liposome containing phytosterol to a subject in an amount effective for inhibiting cancer metastasis. The present invention additionally relates to the use of a phytosterol-containing liposome for the prevention and treatment of cancer. There are no limitations here on the type of cancer, and the cancer can be exemplified by head and neck cancers, stomach cancer, colon cancer, rectal cancer, colorectal cancer, liver cancer, gall bladder · bile duct cancers, pancreatic cancer, lung cancer, breast cancer, bladder cancer, prostate cancer, uterine cancer, oral cancer, pharyngeal cancer, throat cancer, tongue cancer, esophageal cancer, renal cancer, ovarian cancer, and so forth. This phytosterol-containing liposome is particularly effective against mucous membrane cancers and can therefore be expected to be highly effective against colorectal cancer, oral cancer, tongue cancer, throat cancer, and lung cancer.

A liposome is a lipid vesicle having one or more bimolecular membranes formed by the hydration with an adequate amount of water of lipid comprising mainly phospholipid. Liposomes are currently classified based on the number of lipid bilayers and are classified into multilamellar liposomes (MLV) and unilamellar liposomes. The unilamellar liposomes are classified by size into small unilamellar vesicles (SUV), large unilamellar vesicles (LW), and giant unilamellar vesicles (GUV). The liposome of the present invention may be any of these. The liposome of the present invention is preferably an MLV. The size of the liposome in the present invention is generally 30 to 1000 nm, preferably 30 to 600 nm, more preferably 50 to 400 nm, and even more preferably 50 to 200 nm.

The liposome can be produced by known methods. For example, prescribed amounts of lecithin and sterol can be solubilized in a suitable organic solvent, for example, ethanol; the membrane lipid can be produced by removing the solvent under reduced pressure; an aqueous solution comprising, for example, any of various buffers can be added thereto; and a liposome suspension can be prepared by stirring, for example, for about 2 to 5 minutes at about 1000 to 3000 rpm.

As necessary, the obtained suspension can be subjected to a process that removes physiologically active substance present in the liquid outside the liposome; for example, a process can be carried out in which the suspension is filtered and the resulting filtrate is then subjected to dialysis.

Phytosterol is used as the sterol in the present invention. Phytosterol is known to occur mainly as constituent components of plant cells. The phytosterol can be exemplified by β-sitosterol, campesterol, stigmasterol, brassicasterol, ergosterol, ergostadienol, and so forth. A single phytosterol can be used or two or more phytosterols can be used in combination. The phytosterol is preferably β-sitosterol. The phytosterol content in the liposome is generally 1 to 33 weight%, preferably 10 to 33 weight%, and more preferably 10 to 25 weight%.

The lecithin used in the present invention can be a single selection or a combination of two or more selections from, for example, egg yolk lecithin, soy lecithin, rapeseed lecithin, corn lecithin, sunflower lecithin, peanut lecithin, and so forth, but is not limited to the preceding. The present invention can also use the product of the hydrogenation of the preceding. Lecithin is also known as phosphatidylcholine or 1,2-diacylglycerol-3-phosphocholine and as a general matter contains fatty acid bonded at positions 1 and 2 of glycerol. In addition to the lecithin cited above as examples, the use is preferred in the present invention of lecithin in which $C_{12-24}$ unsaturated fatty acid is bonded at both positions 1 and 2 or at one of positions 1 and 2, while the use is particularly preferred of lecithin that has a $C_{12-24}$ saturated fatty acid bonded at position 1 and a $C_{12-24}$ unsaturated fatty acid bonded at position 2. The saturated fatty acid and unsaturated fatty acid referenced here may be straight chain or branched. The use of such lecithin results in an improved enteric absorption of the physiologically active substance incorporated in the liposome. A $C_{16-18}$ unsaturated fatty acid is preferably used as the unsaturated fatty acid, and an even better enteric absorption can be expected in particular for the use of lecithin that contains large amounts of oleic acid or linoleic acid bonded at position 2. Egg yolk lecithin, soy lecithin, sunflower lecithin, and rapeseed lecithin are preferred for the present invention. The lecithin content of the liposome is generally 67 to 99 weight%, preferably 67 to 90 weight%, and more preferably 75 to 90 weight%.

The lecithin : phytosterol molar ratio in the liposome in the present invention is preferably about 50 : 50 to 90 : 10, more preferably about 55 : 45 to 85 : 15, and most preferably 60 : 40 to 70 : 30. The stability of the liposome membrane is improved when the molar ratio is in these ranges.

The lecithin content and sterol content can be measured by already known methods. For example, the lecithin content can be determined by the Fiske-Subbarow method, while the sterol content can be determined by, for example, HPLC or colorimetry.

The surface of the liposome can be coated, and this coated-liposome can also be utilized as an effective component. Coating by a sulfated polysaccharide is an example of a preferred coating. This sulfated polysaccharide can be exemplified by fucoidan, carrageenan, agar, heparin, and so forth. This sulfated polysaccharide also encompasses products obtained by the sulfation of polysaccharide lacking sulfate groups, for example, chondroitin sulfate, dermatan sulfate, and so forth.

The use of sulfated polysaccharide having a molecular weight of about 5000 to 300,000 is preferred. Among these sulfated polysaccharides, the use of fucoidan and carrageenan is preferred and fucoidan is particularly preferred.

The sulfated polysaccharide is used, for example, preferably at about 10 to 500 weight parts and more preferably at about 20 to 200 weight parts, in each case per 100 weight parts of the lecithin present in the liposome.

Coating can be carried out, for example, by adding a sulfated polysaccharide to the suspension of the phytosterol-containing liposome and stirring for about 2 to 5 minutes at about 1000 to 3000 rpm. A plurality of liposomes may also be contained in 1 coated membrane.

That the liposome is coated by the sulfated polysaccharide can be confirmed, for example, by the fact that the zeta potential of the liposome solution is changed by the addition of the sulfated polysaccharide and stirring.

In addition to lecithin and phytosterol, for example, the following can be added to the liposome as necessary: antioxidants such as α-tocopherol, ascorbic acid, and so forth; organic acids such as lactic acid, citric acid, and so forth; lipids such as phosphatidylglycerol, phosphatidylethanolamine, and so forth; natural polymers such as chitosan, fucoidan, hyaluronic acid, and so forth; synthetic polymers such as polyethylene glycol, carboxyvinyl polymers, and so forth; sugars such as trehalose, lactulose, maltitol, and so forth; and polyol such as glycerin, and so forth.

A variety of substances can be incorporated on an optional basis as contents of the liposome used in the present invention. Examples are therapeutically effective substances such as doxorubicin, daunorubicin, cisplatin, taxol, and so forth, and components that have a cancer-suppressing effect, such as β-glucan, polysaccharides, and so forth. The liposome that entraps a content material can be produced by heretofore known methods. For example, specified quantities of lecithin and phytosterol can be solubilized with a suitable solvent, for example, ethanol; the membrane lipid can be prepared by removing the solvent under reduced pressure; an aqueous solution containing the content material can be added thereto; and a liposome suspension can then be obtained by stirring, for example, at about 1000 to 3000 rpm for about 2 to 5 minutes. The quantity of content material encompassed by the liposome is generally 1 to 1000 weight% with reference to the lipid in liposome and is preferably 10 to 500 weight% with reference to the lipid in the liposome.

As necessary, the obtained suspension can be subjected to a process that removes the content material present in the liquid outside the liposome; for example, a process can be carried out in which the suspension is filtered and the resulting filtrate is then subjected to dialysis.

When the orally administered composition of the present invention is used in the form of a food composition, this can be prepared by the usual methods, as necessary combining the liposome with, for example, an edible vehicle, a food ingredient, a food additive, and so forth, adapted to the form of the food. The food can take the form of a liquid food, such as a beverage; a solid food, such as a tablet, granule, or chewable tablet; and so forth. Semi-solid foods can also be used, for example, yogurt. The form of the food can be specifically exemplified by liquid beverages such as juices, soft drinks, teas, and so forth; powdered beverages such as powdered juices, powdered soups, and so forth; confections and sweets such as chocolates, candies, chewing gum, ice cream, jellies, cookies, biscuits, corn flakes, chewable tablets, film sheet confections, wafers, gummi candies, senbei (Japanese rice cracker), manju (sweet bean paste bun), and so forth; condiments such as dressings, sauces, and so forth; and also breads, noodles, konnyaku, boiled fish paste products (e.g., kamaboko and so forth), furikake (a seasoned powder for sprinkling over rice), oral sprays, lozenges, and so forth. Additives may be admixed, for example, live or killed bacteria such as lactic acid bacteria, other probioactive substances, vitamins, herbal and natural medicines, and plants, such as herbs, either as such or in the form of an extract.

The vehicle in the food composition can be exemplified by sugar alcohols such as maltitol, xylitol, sorbitol, erythritol, and so forth; fillers and diluents such as crystalline cellulose, lactose, sucrose, glucose, starch, carbonates, phosphates, and so forth; binders such as gelatin, alginic acid, xanthan gum, cellulose, hydroxypropyl cellulose, methyl cellulose, carrageenan, pullulan, pectin, and so forth; emulsifying agents such as sucrose fatty acid esters, sorbitan fatty acid esters, enzymatically treated lecithin, enzymatically digested lecithin, saponin, and so forth; antioxidants such as ascorbic acid, tocopherol, and so forth; acidulants such as lactic acid, citric acid, gluconic acid, glutamic acid, and so forth; fortifying agents such as vitamins, amino acids, lactate, citrate, gluconate, and so forth; fluidizers such as silicon dioxide and so forth; lubricants such as sucrose fatty acid esters, stearates, and so forth; oligosaccharides such as lactulose, raffinose, fructooligosaccharide, isomaltooligosaccharide, xylooligosaccharide, soy oligosaccharide, and so forth; dietary fiber such as indigestible dextrin, psyllium, beet fiber, and so forth; sweeteners such as sucralose, acesulfame potassium, aspartame, glycyrrhizin, and so forth; and flavorants such as peppermint oil, eucalyptus oil, cinnamon oil, fennel oil, clove oil, orange oil, lemon oil, rose oil, fruit flavor, mint flavor, peppermint powder, dl-menthol, 1-menthol, and so forth. Additives may be admixed, for example, live or killed bacteria such as lactic acid bacteria, other probioactive substances, prebiotics, vitamins, herbal and natural medicines, and plants, such as herbs, either as such or in the form of an extract.

The oral food composition of the present invention can be used for applications such as a health food, functional food, Food for Specified Health Use, Food with Nutrient Functional Claims, Medical Food for the Ill, and so forth, in each case having, for example, an inhibitory activity on cancer metastasis, a cancer preventive activity, or a cancer treatment activity.

For the oral food composition of the present invention, the liposome content with reference to the total quantity of the oral food composition is set as appropriate considering, for example, the form of the composition, and is generally 0.5 to 95 weight%, preferably 0.8 to 80 weight%, and more preferably 1 to 50 weight%. In those instances where the composition of the present invention is a beverage, an even more preferred range for the liposome content is 1 to 4 weight%.

The orally administered composition of the present invention contains the phytosterol-containing liposome and as necessary a pharmaceutically acceptable vehicle and can be used as an orally administratable pharmaceutical composition, for example, a liquid formulation; a solid formulation such as a tablet, granular formulation, fine granule, powder, and so forth; or a capsule containing the aforementioned liquid or solid formulation. The pharmaceutically acceptable vehicle can be exemplified by fillers, diluents, and so forth. The oral pharmaceutical composition can also contain various additives, for examples, flavorants and so forth.

The vehicles and additives can be exemplified by the following: fillers and diluents such as sugar alcohols (e.g., maltitol, xylitol, sorbitol, erythritol, and so forth), lactose, sucrose, sodium chloride, glucose, starch, carbonates (e.g., calcium carbonate and so forth), kaolin, crystalline cellulose, silicic acid, methyl cellulose, glycerol, sodium alginate, gum arabic, talc, phosphates (e.g., calcium monohydrogen phosphate, calcium hydrogen phosphate, sodium hydrogen phosphate, dipotassium phosphate, potassium dihydrogen phosphate, calcium dihydrogen phosphate, sodium dihydrogen phosphate, and so forth), calcium sulfate, calcium lactate, cacao butter, and so forth; binders such as simple syrup, glucose solutions, starch solutions, gelatin solutions, polyvinyl alcohol, polyvinyl ether, polyvinylpyrrolidone, crosslinked polyvinylpyrrolidone, hydroxypropyl cellulose, low-substituted hydroxypropyl cellulose, hydroxypropyl methyl cellulose, hydroxyethyl cellulose, carboxylvinyl polymers, crystalline cellulose, powdered cellulose, crystalline cellulose sodium carmelose, carboxymethyl cellulose, shellac, methyl cellulose, ethyl cellulose, potassium phosphate, powdered gum arabic, pullulan, pectin, dextrin, corn starch, pregelatinized starch, hydroxypropyl starch, gelatin, xanthan gum, carrageenan, tragacanth, powdered tragacanth, macrogol, and so forth; disintegrants such as dried starch, sodium alginate, powdered agar, powdered laminaran, sodium bicarbonate, calcium carbonate, polyoxyethylene sorbitan fatty acid esters, sodium lauryl sulfate, stearic acid monoglyceride, starch, lactose, and so forth; disintegration inhibitors such as sucrose, stearic acid, cacao butter, hydrogenated oil, and so forth; absorption promoters such as quaternary ammonium salts, sodium lauryl sulfate, and so forth; humectants such as glycerol, starch, and so forth; adsorbents such as starch, lactose, kaolin, bentonite, colloidal silicic acid, and so forth; lubricants such as purified talc, stearic acid salts, powdered boric acid, polyethylene glycol, and so forth; emulsifying agents such as sucrose fatty acid esters, sorbitan fatty acid esters, enzymatically treated lecithin, enzymatically digested lecithin, saponin, and so forth; antioxidants such as ascorbic acid, tocopherol, and so forth; acidulants such as lactic acid, citric acid, gluconic acid, glutamic acid, and so forth; fortifying agents such as vitamins, amino acids, lactate, citrate, gluconate, and so forth; fluidizers such as silicon dioxide and so forth; sweeteners such as sucralose, acesulfame potassium, aspartame, glycyrrhizin, and so forth; and flavorants such as peppermint oil, eucalyptus oil, cinnamon oil, fennel oil, clove oil, orange oil, lemon oil, rose oil, fruit flavor, mint flavor, peppermint powder, dl-menthol, 1-menthol, and so forth.

Solid forms such as tablets may as necessary take the form of tablets on which the usual coatings have been executed, for example, a sugar-coated tablet, gelatin-coated tablet, enteric-coated tablet, film-coated tablet, tablet-within-a-tablet, multilayer tablet, and so forth. Capsules can also be used in which the liposome is filled into, for example, a hard gelatin capsule, a soft capsule, and so forth.

The liquid formulations may be aqueous or oil-based suspensions, solutions, syrups, or elixirs and can be prepared according to the usual methods using, for example, the usual vehicles, additives, and so forth.

The pharmaceutical composition can be used as an anticancer agent, an agent that inhibits cancer metastasis, or a cancer preventive.

For the oral pharmaceutical composition of the present invention, the liposome content with reference to the total quantity of the oral pharmaceutical composition is set as appropriate considering, for example, the form of the composition, and is generally 0.5 to 95 weight%, preferably 0.8 to 80 weight%, and more preferably 1 to 50 weight%. In those instances where the composition of the present invention is a liquid formulation, an even more preferred range for the liposome content is 1 to 4 weight%.

With regard to the amount of intake of the orally administered composition of the present invention, the quantity of liposome intake per adult per day is generally 10 mg to 10000 mg, preferably 20 mg to 2000 mg, and more preferably 50 mg to 2000 mg.

EFFECT OF THE INVENTION

The phytosterol-containing liposomes have the ability to significantly inhibit cancer metastasis. The present invention is useful for inhibiting cancer metastasis and for the prevention and treatment of cancer. The orally administered composition of the present invention is useful in particular as a food composition and as a pharmaceutical composition.

BEST MODE FOR CARRYING OUT THE INVENTION

The present invention is described in greater detail by the examples and test examples shown below; however, the present invention is not limited to these examples.

EXAMPLES

Example 1

Preparation of liposomes in which β-sitosterol and egg yolk lecithin are membrane constituent components

Liposomes were produced by the thin film hydration method. 160 $\mu$L of a solution of egg yolk lecithin dissolved at a concentration of 500 mM in chloroform and 160 $\mu$L of a solution of β-sitosterol dissolved at a concentration of 250 mM in chloroform were measured out and the two solutions were mixed, giving an egg yolk lecithin : β-sitosterol molar ratio of 2 : 1, and chloroform was then added. The chloroform was distilled off under reduced pressure on a rotary evaporator followed by drying under a vacuum to produce a thin film. 4 mL phosphate-buffered physiological saline (PBS) was added to this thin film to bring the egg yolk lecithin concentration to 20 mM and a liposome suspension was obtained by processing with a vortex mixer and carrying out three freeze-thaw cycles with liquid nitrogen. This suspension was treated for 10 minutes with a probe-type sonicator to yield a liposome composition.

Comparative Example 1

Preparation of liposomes in which cholesterol and egg yolk lecithin are membrane constituent components

A liposome composition was obtained as in Example 1, except that in this case the β-sitosterol was replaced with cholesterol.

Test Example 1

Metastasis-inhibiting activity in B16BL6 melanoma cells by the liposome compositions

Twenty 5-week old C57BL6/J mice were divided into four groups. The control group received 0.2 mL phosphate-buffered physiological saline (PBS) once per day by forced oral administration up to and including the 6th day of keeping. In like manner, the liposome composition of Example 1 was orally administered instead of the PBS to the group designated as the liposomized β-sitosterol group; an aqueous suspension of ordinary, unliposomized β-sitosterol (contained the same amount of β-sitosterol as the liposomized β-sitosterol administered to the liposomized β-sitosterol group) was orally administered instead of the PBS to the group designated as the β-sitosterol group; and the liposome composition of Comparative Example 1 was orally administered instead of the PBS to the group designated as the liposomized cholesterol group. B16BL6 melanoma cells suspended in serum-free medium were administered intravenously ($3 \times 10^4$/0.2 ml/animal) on the 7th day of keeping. The mice were dissected on the 21st day of keeping and the lungs were extirpated; the lung surfaces were inspected and the number of colonies was counted. The average number of colonies is shown in Table 1 for each group.

Table 1.

| | Liposomized β-sitosterol group | β-Sitosterol group | Liposomized cholesterol group | Control group |
|---|---|---|---|---|
| Average number of colonies | 22 | 43 | 51 | 66 |

The liposomes in which β-sitosterol was a membrane constituent component significantly inhibited tumor cell metastasis as compared with unliposomized β-sitosterol and the liposomes in which cholesterol was a membrane constituent component. This demonstrated that phytosterol-containing liposomes are effective against solid cancers, such as colorectal cancer, oral cancer, stomach cancer, pharyngeal cancer, lung cancer, and so forth. Phytosterol-containing liposomes are therefore useful as the effective component of an orally administered anticancer composition.

Test Example 2

Effect of the liposome compositions on NK cells

Three 5-week old C57BL6/J mice were divided into three groups. The control group received 0.2 mL phosphate-buffered physiological saline (PBS) once per day by forced oral administration up to and including the 6th day of keeping. In like manner, the liposome composition of Example 1 was orally administered instead of the PBS to the group designated as the phytosterol group and the liposome composition of Comparative Example 1 was orally administered instead of the PBS to the group designated as the cholesterol group. On the 7th day of keeping the mice were dissected and the spleen was extirpated under sterile conditions and individual spleen cell suspensions were prepared. YAC-1 cells (target cells) were labeled with $Na_2^{51}CrO_4$ (3.7 MBq/$10^6$ cells). 100 μL of the labeled YAC-1 cells ($1 \times 10^4$/well) was introduced into a 96-well titer plate, and to the wells was added, so as to give a spleen cell (effector cell, NK cell) : YAC-1 cell ratio (E/T ratio) of 50 : 1 or 25 : 1, 100 μL NK cells (NK cell-added wells). Wells were also prepared without the addition of the NK cells (non-NK cell well). Triton X-100 was added to wells (Triton-added wells) in which the cell type, cell quantity, and cell ratio were the same as in the NK cell-added wells. This plate was incubated for 4 hours at 37°C in a 5% $CO_2$ atmosphere followed by centrifugal separation for 5 minutes at 200g. The liberated radioactivity in the supernatant yielded by centrifugal separation was measured with a gamma counter. The $^{51}$cr release ratio (NK activity) was calculated with the following equation.

$$\text{release ratio (\%)} = \frac{(\text{measured value for NK cell-added wells} - \text{measured value for non-NK cell wells})}{(\text{measured value for Triton-added wells} - \text{measured value for non-NK cell wells})} \times 100$$

The measured value for the Triton-added well, which was obtained by the addition of Triton X-100 to the target cells (YAC-1 cells) and effector cells (NK cells), is the maximum release value and is the amount of Cr label released upon the disruption of all the cancer cells and thus corresponds to the total number of cancer cells. The measured value for the NK cell-added wells is the amount of Cr label released upon the addition of NK cells and as such corresponds to the number of cancer cells killed by the NK cells. The measured value for the non-NK cell wells is the control and is the amount of Cr label released when only the YAC-1 cells are present.

The results are shown in Figure 1. While the cholesterol group exhibited a higher NK activity than the control group, the phytosterol group exhibited a significantly higher NK activity than even this cholesterol group.

Formulation Example 1: Tablets

Liposomes were prepared using egg yolk lecithin and phytosterol, and tablets with the following composition were produced by a standard method using these liposomes and the other components.

| | |
|---|---|
| egg yolk lecithin | 4.0 weight% |
| phytosterol | 1.0 weight% |
| maltitol | 42.0 weight% |
| lactose | 25.0 weight% |
| lactulose | 20.0 weight% |
| sucrose fatty acid ester | 8.0 weight% |

Formulation Example 2: Granules

Liposomes were prepared using egg yolk lecithin and phytosterol, and granules with the following composition were produced by a standard method using these liposanes and the other components.

| | |
|---|---|
| egg yolk lecithin | 5.0 weight% |
| phytosterol | 1.0 weight% |
| lactose | 41.3 weight% |
| starch | 50.0 weight% |
| xanthan gum | 2.0 weight% |
| tocopherol | 0.1 weight% |
| gluconic acid | 0.1 weight% |
| flavorant | 0.5 weight% |

Formulation Example 3: Beverage

Liposomes were prepared using egg yolk lecithin and phytosterol, and a beverage with the following composition was produced by a standard method using these liposomes and the other components.

| | |
|---|---|
| egg yolk lecithin | 1.0 weight% |
| phytosterol | 0.2 weight% |
| citric acid | 0.5 weight% |
| sodium citrate | 0.5 weight% |
| high fructose corn syrup | 10.0 weight% |
| vitamin C | 0.5 weight% |
| flavorant | 0.15 weight% |
| purified water | remainder |

**Claims**

1. Phytosterol for use in the prevention or treatment of cancer, wherein the phytosterolis contained in a liposome.

2. The phytosterol for use according to claim 1, wherein the phytosterol is at least one selected from the group consisting of β-sitosterol, campesterol, stigmasterol, brassicasterol, ergosterol, and ergostadienol.

3. The phytosterol for use according to claim 1, wherein the phytosterol content of the liposome is 1 to 33 weight%.

4. The phytosterol for use according to any of claims 1 to 3, wherein the anticancer activity is an inhibition of cancer metastasis.

5. The phytosterol for use according to any of claims 1 to 4, wherein the amount of liposome that is orally administered is 10 mg to 10000 mg per adult per day.

6. The phytosterol for use defined according to any of claims 1 to 5, wherein the liposome is comprised in a food composition.

7. The phytosterol for use defined according to any of claims 1 to 5, wherein the liposome is comprised in a pharmaceutical composition.

**Patentansprüche**

1. Phytosterol für die Verwendung in der Vorbeugung oder der Behandlung von Krebs, wobei das Phytosterol in einem Liposom enthalten ist.

2. Phytosterol für die Verwendung nach Anspruch 1, wobei das Phytosterol wenigstens eines ausgewählt aus der Gruppe bestehend aus β-Sitosterol, Campesterol, Stigmasterol, Brassicasterol, Ergosterol und Ergostadienol ist.

3. Phytosterol für die Verwendung nach Anspruch 1, wobei der Phytosterolgehalt des Liposoms 1 bis 33 Gewichts-% ist.

4. Phytosterol für die Verwendung nach einem der Ansprüche 1 bis 3, wobei die Antikrebswirkung eine Hemmung der Krebsmetastasierung ist.

5. Phytosterol für die Verwendung nach einem der Ansprüche 1 bis 4, wobei die Menge an Liposom, die oral verabreicht wird, von 10 mg bis 10000 mg pro Erwachsener pro Tag ist.

6. Phytosterol für die Verwendung definiert nach einem der Ansprüche 1 bis 5, wobei das Liposom in einer Lebensmittelzusammensetzung umfasst ist.

7. Phytosterol für die Verwendung definiert nach einem der Ansprüche 1 bis 5, wobei das Liposom in einer pharmazeutischen Zusammensetzung umfasst ist.

**Revendications**

1. Phytostérol destiné à être utilisé dans la prévention ou le traitement du cancer, dans lequel le phytostérol est contenu dans un liposome.

2. Phytostérol destiné à être utilisé selon la revendication 1, dans lequel le phytostérol est au moins un phytostérol choisi dans le groupe constitué du β-sitostérol, du campestérol, du stigmastérol, du brassicastérol, de l'ergostérol, et de l'ergostadiénol.

3. Phytostérol destiné à être utilisé selon la revendication 1, dans lequel la teneur en phytostérol du liposome est de 1 à 33 % en poids.

4. Phytostérol destiné à être utilisé selon l'une quelconque des revendications 1 à 3, dans lequel l'activité anticancéreuse est une inhibition de la métastase du cancer.

**5.** Phytostérol destiné à être utilisé selon l'une quelconque des revendications 1 à 4, dans lequel la quantité de liposome qui est administrée par voie orale est de 10 mg à 10 000 mg par adulte par jour.

**6.** Phytostérol destiné à être utilisé selon l'une quelconque des revendications 1 à 5, dans lequel le liposome est compris dans une composition alimentaire.

**7.** Phytostérol destiné à être utilisé selon l'une quelconque des revendications 1 à 5, dans lequel le liposome est compris dans une composition pharmaceutique.

FIGURE 1.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004010977 A

- WO 03039437 A

### Non-patent literature cited in the description

- **HIROFUMI TAKEUCHI et al.** Enteral Absorption of Insulin in Rats from Micoadhesive Chitosan-Coated Liposanes. *Pharmaceutical Research,* 1996, vol. 13 (6), 896-901
- **MURAMATSU, K. et al.** Dipalmitoylphosphatidylcholine Liposomes with Soybean-Derived Sterols and Cholesterol as a Carrier for the Oral. Administration of Insulin in Rats. *Biological & Pharmaceutical Bulletin,* 1996, vol. 19 (8), 1055-1058
- **KAZUNORI IWANAGA.** Application of surface-coated liposomes for oral delivery of peptide: Effects of coating the liposome's surface on the GI transit of insulin. *Journal of Pharmaceutical Sciences,* 1999, vol. 88, 248-252

- **YOSHIE MAITANI et al.** Oral administration of recombinant human erythropoietin in liposomes in rats: Influence of lipid composition and size of liposomes on bioavailability. *Journal of Pharmaceutical Sciences,* 1996, vol. 85, 440-445
- **SHIMIZU K et al.** BIOLOGICAL & PHARMACEUTICAL BULLETIN. PHARMACEUTICAL SOCIETY OF JAPAN, vol. 21, 741-746
- **AWAD A B et al.** EUROPEAN JOURNAL OF CANCER PREVENTION. LIPPINCOTT WILLIAMS & WILKINS, vol. 10, 507-513
- **MAITANI Y et al.** CURRENT PHARMACEUTICAL BIOTECHNOLOGY. BENTHAM SCIENCE PUBLISHERS, vol. 6, 81-93